# EUROPEAN PATENT SPECIFICATION

(11) **EP 1 764 128 B1**
(45) Date of publication and mention of the grant of the patent: **14.07.2010**
(21) Application number: 06111309.8
(22) Date of filing: 17.03.2006
(51) Int. Cl.: A61M 29/02, A61M 25/10, A61F 2/00

(54) **Device for cellular therapy**
Vorrichtung für die Zelltherapie
Dispositif pour la thérapie cellulaire

(30) Priority: 20.09.2005 IT TO20050650
(43) Date of publication of application: 21.03.2007
(73) Proprietor: Sorin Biomedica Cardio S.r.l., 13040 Saluggia (Vercelli) (IT)
(72) Inventor: Vallana, Franco, 10123 Torino (IT); Rolando, Giovanni, 10034 Chivasso (Torino) (IT); Curcio, Maria, 13040 Saluggia (Vercelli) (IT); Grignani, Andrea, 10023 Chieri (Torino) (IT)
(74) Representative: Bosotti, Luciano

(56) References cited:
- EP-A2- 1 181 903
- WO-A-00/41761
- WO-A2-99/16500
- US-A- 5 397 307
- US-A1- 2002 077 594
- US-A1- 2004 073 296

## Description

The present invention relates to techniques for cellular therapy, and has been developed with particular attention to its possible application to treating the cardiac muscle.

Such techniques entail the utilisation of cells that, distributed in a cardiac site, are able to "repair" the site itself, for example repairing parts of the damage suffered by the cardiac muscle due to an ischaemic event (infarction). The cells in general are different cells from those that constitutes the cardiac muscle. In most cases they are autologous cells, that is from the patient him/herself, such as stem cells.

Although these are rather recent techniques, there is considerable literature on the question, as is shown by the following documents:
- P. Menasché et al., «Myoblast transplantation for heart failure», Lancet 357, 279-280, 27 gennaio, 2001;
- P. Menasché et al., «Doctor Puts Arm Muscle Cells Into Patient's Heart», Associated Press, 30 maggio 2001;
- P. Menasché et al., «First Percutaneous Endovascular Case of Heart Muscle Regeneration Completed with Bioheart's MyoCell (TM) Product», PRNewswire, 30 maggio 2001;
- R.M. El Oakley et al., «Myocyte transplantation for cardiac repair: A few good cells can mend a broken heart", Annals of Thoracic Surgery 71, 1724 - 1733, 2001;
- K. A. Jackson et al.: «Regeneration of ischemic cardiac muscle and vascular endothelium by adult stem cells», Journal of Clinical Investigation 107, 1395 - 1402, giugno 2001;
- N. N. Malouf et al., «Adult-derived stem cells from the liver become myocytes in the heart in vivo", American Journal of Pathology 158, 1929 - 1935, giugno 2001;
- D. Orlic et al., «Bone marrow cells regenerate infarcted myocardium», Nature 410, 701 - 705, 5 aprile 2001;
- A. A. Kocher et al., «Neovascularization of ischemic myocardium by human bone-marrow-derived angioblasts prevents cardiomyocyte apoptosis, reduces remodeling and improves cardiac function», Nature Medicine 7, 430-436, aprile 2001;
- J. S. Wang et al., «Marrow stromal cells for cellular cardiomyoplasty: feasibility and potential clinical advantages», The Journal of Thoracic and Cardiovascular Surgery 120, 999-1006, novembre 2000;
- M. Scorsin et al., « Comparison of the effects of fetal cardiomyocyte and skeletal myoblast transplantation on postinfarction left ventricular function», The Journal of Thoracic and Cardiovascular Surgery 119, 1169-1175, giugno 2000; e
- T. Siminiak et al., «Myocardial Replacement Therapy», Circulation 2003; 108: 1167-1171.

In general, dissemination of cells in cardiac cellular therapy comes about by injection into the cardiac muscle, for example during heart surgery, or in a cardiological environment, utilising an intra-coronary catheter.

Although such treatment is undoubtedly promising, the results achieved to date cannot be considered entirely satisfactory.

Tests have shown, for example, that there is a risk of generating arrhythmia or of achieving results that are overall poor. In particular, for example, the final destiny of the cells used for therapy is not entirely clear nor under control. More precisely -- in the opinion of some researchers -- in the case of stem cells the cells themselves might undesirably reach sites other than the treatment site, and stimulate aberrant or in any case undesirable cell growth.

At least during these early years, the attention of researchers and experimenters appears to have been concentrated primarily on the characteristics of the cellular material utilised for therapy.

A significant line of research has been aimed at identifying which cells are preferable, as a function of the type of therapy. At present, the prevailing tendency is to prefer stem cells, harvested for example from the bone marrow, rather than muscular cells, of skeletal origin.

Another line of investigation is linked to possible treatments that the cells undergo after harvesting in view of their utilisation: the cells in fact may be harvested and caused to grow in vitro in view of their use for therapy, or alternatively implanted immediately after harvesting.

Another extensive line of research is linked to the possibility of associating chemical substances such as for example cytokines to the cells used for treatment. It is indeed thought that cytokines are able to stimulate the stem cells present in the heart.

One of the major problems of achieving truly satisfactory effects is connected with the survival of cells in the treated tissue; the possibilities offered by associating drugs or growth factors to the cells are interesting from this standpoint. This is also true with regard to the possible addition to the cells of bio-materials able to ensure survival of the implanted cells.

Overall, less attention has been paid to date to the specific modalities of implantation.

The application through the distal portion of a catheter introduced into the patient's body through the peripheral vascular system (for example through the femoral artery), thus with an essentially non-invasive method, appears greatly preferable over direct injection into the heart performed during heart surgery.

The present invention has the object of providing a improved device for the performance of cellular therapy, such as for example cardiac cellular therapy. More specifically, the invention relates to a device having the features set forth in the preamble of claim 1, which is known i.a. from US-A-5 397 307.

According to the present invention, this object is achieved thanks to a device having the further features set forth in the characterizing portion of claim 1.

The claims form an integral part of the disclosure of the invention provided herein.

The embodiment of the invention that is preferred at present is a device for cellular therapy suitable for introduction through a catheter towards a treatment site (V), the device in question comprising an expandable portion, capable of being expanded between a contracted position and a deployed position at the treatment site, such as to perform an angioplasty treatment at said site, and a conveyor portion, capable of receiving cells for cellular therapy and of delivering said cells at said treatment site concomitantly with the performance of said angioplasty operation.

Preferably, the conveyor portion:
-- is coupled to a supply line for supplying cells for cellular therapy towards said conveyor portion; and/or
-- contains a charge comprising cells for cellular therapy.

Substantially speaking, the device according to the invention makes it possible to perform, simultaneously, during a non-invasive intervention (that is through a catheter) two concomitant actions on a treatment site (typically a blood vessel):
-- implantation on site of the cells destined to achieve the therapy, and
-- an angioplasty treatment, with an effect antagonistic to the stenosis of the treated vessel.

Without intending to restrict this application to any specific theory in this connection, the applicant has reason to believe that the quality of the results achievable with the device according to the invention is linked to the fact that the device makes it possible to achieve revascularisation and to apply a regenerative tissue stimulus concomitantly.

All of this operating in such a way that the action of implanting the cells at the treated site is achieved in a selective fashion. All of this operating such that virtually all the cells used for treatment purposes are targeted towards the treated tissue and not dispersed in the blood stream. In this way the risk is avoided that these dispersed cells are lost for the purposes of achieving the treatment and -- in some cases - are capable of inducing aberrant growth in other districts of the patient's body.

Again, the applicant has reason to believe that the concomitant performance of an angioplasty operation and of the implantation of cells with therapeutic effect means that the treated tissue receives the cells in a condition in which the tissue itself (typically a blood vessel, such as a coronary vessel) is maintained in a condition of at least slight expansion. This in a situation in which the vessel wall is at least partially extended with regard to its normal physiological dimensions. Although it is not at present possible to demonstrate this fact in absolute terms, it may be hypothesised that the greater efficacy of the treatment is linked to the fact that -- in the above extended condition -- the vessel wall is more easily permeable with regard to the implanted cells.

The invention will now be described, as a simple example without limiting intent, with reference to the attached drawings, in which:
-- figure 1 illustrates a first possible embodiment of the device described here,
-- figures 2 illustrates a possible varied embodiment of the same device,
-- figure 3 illustrates the device in figure 1 in conditions of use,
-- figure 4 is a section along the line by IV-IV of figure 3,
-- figure 5 illustrates another possible varied embodiment of the device described here,
-- figure 6 is a section along the line the VI-VI of figure 5.

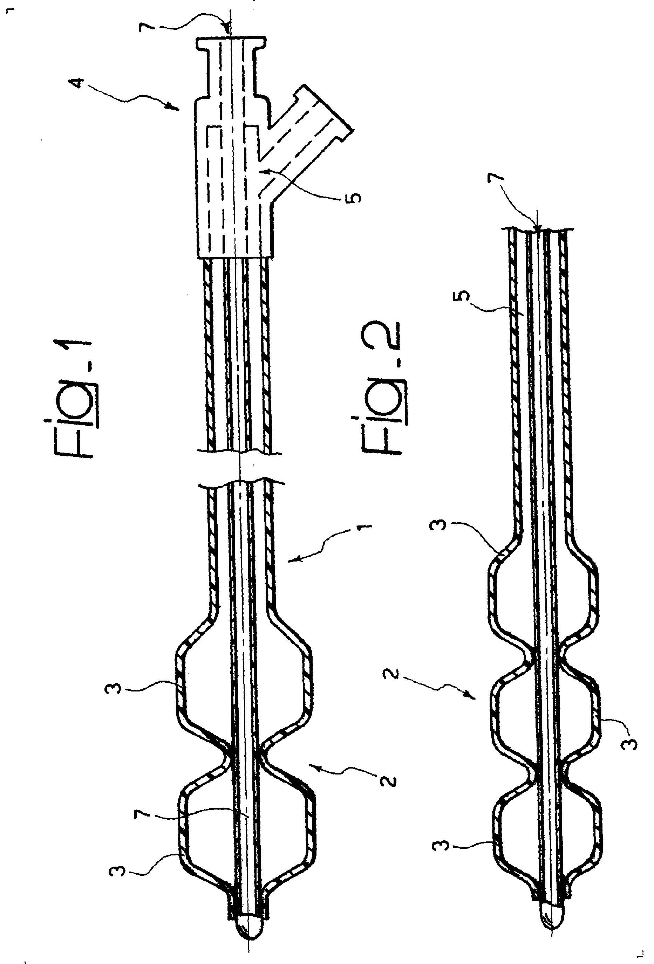

The device illustrated in figures 1 to 4, indicated overall with 1, is configured as a catheter (overall of known type, for which reason it is not specified further here) that in substance can be identified as a "balloon" catheter.

This is a catheter with, at its distal extremity 2, one or more "balloon" elements 3 that can be inflated by means of a fluid made to flow from the proximal extremity 4 (or, in alternative, from an intermediate portion) of the catheter 1 through a tubular cavity 5.

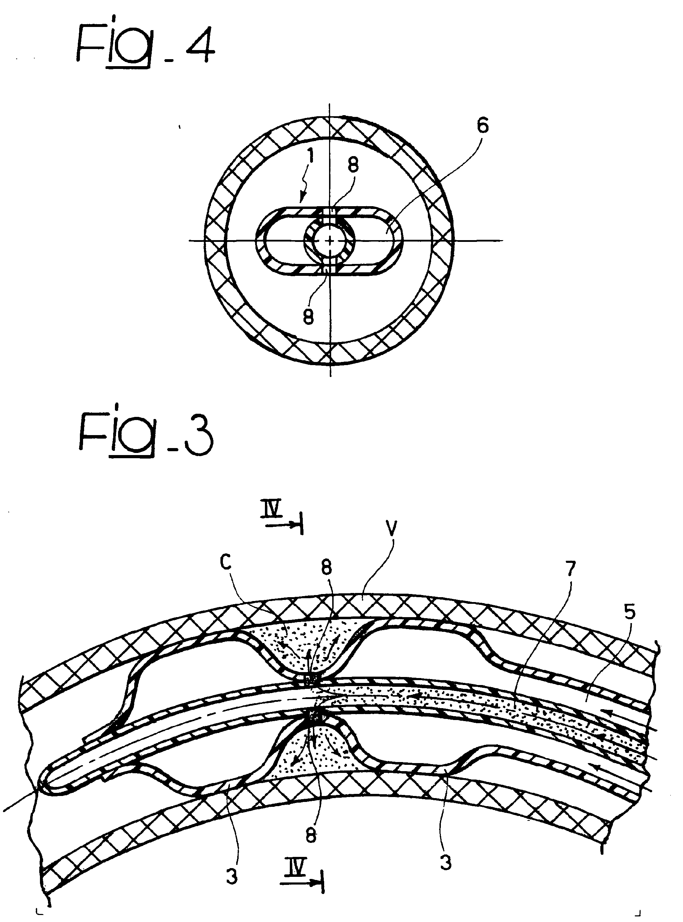

In general the tubular cavity 5 extends to the balloon of the catheter or, in the case -- preferred -- in which more than one balloons 3 are present (for example two or three balloons, as in the devices illustrated in figures 1 and 2) it extends to the balloon situated more distant from the distal extremity of the catheter. In this latter case, the various balloons are in communication (as can better be understood in the section shown in figure 4) through apertures 6 for the passage of the inflation fluid.

For the remaining part, the balloon or balloons 3 present extremities that are narrow, where the balloon or balloons 3 are in close proximity with a central tube 7.

The central tube 7 extends starting from the proximal extremity 4 (or from an intermediate portion) of the catheter and, in correspondence with the zone where the balloon or the balloons 3 are in close proximity with it presents one or more openings 8 (not visible in figures 1 and 2 but clearly visible in figures 3 and 4) communicating with the outside of the catheter 1.

The opening or openings 8 to diffuse the cells C towards the treatment site is/are situated in an adjacent position to the balloon element or elements 3. If only one balloon 3 is present, a preferred embodiment entails the presence of two openings or two groups of openings 8 situated at the two extremities (distal and proximal) of the balloon 3 itself.

If the catheter 1 presents a plurality of balloons (for example two or three, as illustrated in figures 1 and 2) the openings 8 are located in intermediate positions between adjacent balloons 3 -- and of preference only in such intermediate positions. In other words, the openings 8 open onto the outside of the catheter 1 in a zone in any case comprised (in the sense of the longitudinal development of the catheter 1) between adjacent balloons 3.

The materials that can be used to make the central tube 7 and the balloons 3 are those commonly used in catheter technology. Above all in function of the disposable modality of use, plastic materials approved for medical and surgical use are preferred.

The method of use of the device illustrated in figures from 1 to 4 entails that the catheter be introduced into the patient's body in the usual way, for example through the femoral artery, and guided until its distal portion 2 reaches the site to be treated (for example a stenotic coronary vessel V that has caused an ischaemic episode).

Introduction of the catheter comes about with the balloons in the radially retreated condition (that is with the balloons 3 "deflated", in general wrapped around the extremity of the catheter).

Once the treatment site has been reached, the inflation fluid is introduced into the tubular cavity 5 under pressure, such that the balloons 3 inflate and expand radially, in the condition illustrated in figures 1 to 4. In particular, figure 3 shows the angioplastic action performed on the treated vessel, indicated as V, due to radial expansion of the balloons 3.

At the same time, cells C for cellular therapy (such as for example stem cells for cardiac cellular therapy) are introduced -- with known means, for example with a syringe or similar instrument -- into the central tube 7 until they reach the openings 8. From these openings, the cells diffuse outside the catheter 1 thus reaching the walls of the blood vessel V where they perform their therapeutic function.

All of this is concomitant or substantially concomitant with the action of angioplasty deriving from the dilation of the balloons 3.

The fact that the opening or openings 8 are situated in an adjacent position to the balloon or balloons 3 and - for preference - that they open to the outside of the catheter 1 in a zone situated (in the sense of the longitudinal development of the catheter 1) between adjacent balloons 3 means that the cells C are confined in the chamber created between adjacent balloons that, expanding radially, are in contact with the wall of the vessel V, and the wall of the vessel itself, thus avoiding any undesired dispersion.

Naturally, the above-described actions must be put in place in such a fashion as to avoid creating excess pressure capable of having negative effects on the site treated.

For example, after a first radial expansion of a device such as that illustrated in figures 1 and 2, the balloons 3 are at least slightly deflated such as to enable a certain flow of blood from the chamber or chambers created between adjacent balloons into which the cells C have been introduced through the openings 8. The balloons 3 may then again be expanded radially.

As an alternative, said flow may be made possible through grooves provided on the external surface of the balloons 3, extending for preference in a longitudinal direction with regard to the balloons themselves, that is in the general axial direction of the device 1.

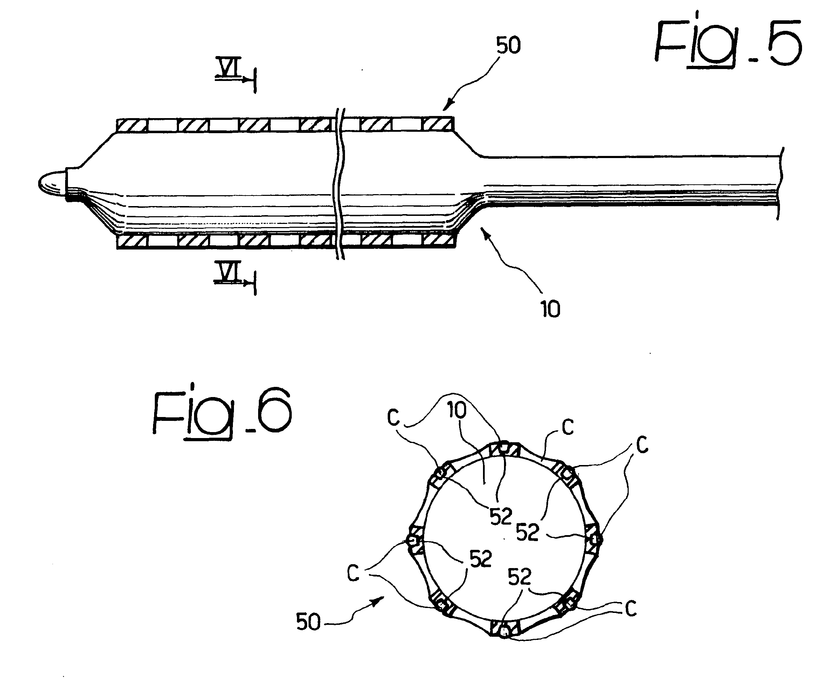

In the embodiment illustrated in figures 5 and 6 the device 1 on the contrary entails the association of a catheter 10 (of known type) to an angioplasty stent indicated overall with 50.

The stent in question may be, for example, of the type described in the documents EP-A-0 850 604, EP-A-1 181 903, EP-A-1 277 449, EP-A-1 310 242, and EP-A-1 449 546, all these documents being held by the present applicant.

The stents in question are angioplasty stents capable of being mounted on the distal extremity of a balloon catheter and of then being positioned at the coronary site following the usual procedures for the use of angioplasty stents. Such modalities of use and implantation are well-known to technology and do not thus require to be described in detail here.

In a further embodiment, not explicitly illustrated in the attached drawings, instead of being of the type expandable through a balloon catheter, the stent 50 may also be of the self-expanding type. This term in general indicates a stent made of super-elastic material (for example the material usually known as Nitinol), also destined to be introduced on site by means of a catheter.

As is well-known to technology, the difference between a stent such as that illustrated in figure 5 and a stent of the self-expanding type lies in the expansion mechanism during the implantation phase.

A stent such as that illustrated in figure 5 is, indeed, destined to be mounted ("crimped" being the usual term) onto the balloon -- maintained "deflated"-- of a balloon catheter. In these conditions, the stent is introduced into the patient's body and made to advance to the site destined to be treated. Having reached the treatment site, the balloon of the catheter is inflated: the fact that the balloon of the catheter is inflated makes the stent 50 pass from the radially contracted condition in which it was crimped onto the balloon to a radially expanded position.

Radial expansion of the stent causes firstly the dilation of the treated vessel, with consequent performance of an angioplasty operation. A second affect lies in the fact that, once expanded radially, the stent 50 conserves its radially-expanded condition (excepting for a slight phenomenon of radial contraction or recoil, induced by reaction against the wall of the treated vessel) thus maintaining the treated vessel in a condition of patency.

In the case of the stent 50 being of the self-expanding type, the distal extremity of the introduction catheter is essentially comprised of a sheath or tunic that initially covers the stent 50 maintaining it in a radially contracted condition. Once the site of implantation has been reached, the said sheath or tunic is retracted in the distal-proximal direction so as to uncover and free the stent. By effect of its super-elastic characteristics (or "shape memory") the stent assumes a radially expanded condition which corresponds - once again - to performing an action of angioplasty and maintaining the treated vessel in a condition of patency.

In both cases, the modalities for production and implantation of the various types of stent considered are of themselves well-known to technology and thus such as not to require a detailed description here.

Whichever type of stent 50 is utilised, the significant characteristic with regard to the device 1 described here is that the stent 50 possesses - for preference only on its radially external surface (that is the surface destined to face towards and be exposed to the wall of the treated vessel V) - hollows 52 in the form of slots, notches, channels or grooves of various types appropriate to receive (if necessary together with other active principles, such as for example drugs contrasting re-stenosis) a charge of cells C for the performance of a cellular therapy, such as cardiac cellular therapy.

In this connection note that, with regard to the solutions described in the last-quoted documents (where the dimensions of the hollows or grooves in the surface are appropriate to receive active principles such as drugs) the hollows 52 considered here must present dimensions compatible with the dimensions of the cells. Substantially, the hollows 52 constitute reservoirs into which the cells C (and any other substances associated with them) are loaded before proceeding to the implantation of the stent.

Loading of the cells into the hollows 52 may come about in different ways. Loading must take into consideration the fact that, in general, it is preferable that the cells C be loaded onto the stent 50 immediately before proceeding to implantation of the stent.

For example, in a particularly simple modality of use, the stent 50 - immediately before being mounted onto the distal extremity of the catheter or, in the case of the catheter of an expandable balloon, also after having been crimped onto the distal extremity of the catheter - is immersed in a mass of treatment cells C in such a fashion that said cells distribute themselves on the external surface of the stent and in particular inside the hollows 52 provided on the external surface of the stent 50.

In this fashion, only that fraction of cells C - many times less numerous - that deposit themselves on the parts of the outer surface of the stent surrounding the hollows or reservoirs 52 becomes dispersed along the penetration route of the catheter. The remainder of the cells (which amply predominate) remain on the contrary inside the hollows or reservoirs 52 and between the mesh, that is between the struts, of the stent 50.

In consequence, when the stent 50 reaches the site of implantation and attains its radially expanded position, the cells C received into the hollows 52 are exposed to the wall of the treated blood vessel V as the sole administration route of the cells towards the treated cardiac site.

Also the cells C that have been received within the openings between the mesh or struts of the stent 50 find themselves in an approximately similar situation since, on the radially inner side of the stent, these openings are closed by the wall of the balloon of the catheter 10. Of note, since the intervention for positioning the stent usually involves expansion of the catheter 10 in several stages, the cells C have the possibility of being absorbed by the treated site before the blood flow is fully restored.

This minimises (and virtually eliminates) the risk of an appreciable fraction of cells being washed away by the blood flow (restored by effect of the angioplasty action) and thus being wasted for treatment purposes.

The high concentration of cells that can be achieved inside the hollows 52 means that the cells themselves may diffuse inside the vessel wall (and hence into the cardiac tissue) with marked penetration efficacy and thus marked treatment efficacy.

In this connection it should be observed that the cells C are in general applied/loaded onto the device 1 as "live" cells. In consequence, when - in the present description and in the attached claims - mention is made of applying and/or loading cells C onto the device 1 it is intended that such application and/or loading usually involves cells C associated with substances (for example, vehicles or matrices, cytokines, growth factors or other substances) destined to ensure the therapeutic efficacy of the cells C themselves.

To advantage, the stent 50 is provided - in whole or in part - with a coating of biocompatible carbon-based material of the type described in documents such as, for example, US-A-5 084 151, US-A-5 133 845, US-A-5 370 684, US-A-5 387 247, or US-A-5 423 886.

The presence of such a coating inside the hollows 52 has been shown to be advantageous since it avoids any undesirable reaction between the cells C (and any substances associated with them) and the material of the stent, preserving the maximum therapeutic activity of the cells C.

The presence of such coating on the inner surface of the stent 50 has been shown to be advantageous since it contrasts undesirable phenomena of coagulation/thrombosing.

The choice of providing the stent 50 with such a coating on its entire surface or only on the radially external surface or only on the radially internal surface is thus dictated by specific application requirements.

Naturally, the principle of the invention holding good, production details and embodiments may vary widely with regard to what is described and illustrated here, without thereby departing from the sphere of the present invention, as defined by the attached claims.

In particular, it will be appreciated that the examples described and illustrated above correspond to situations in which the conveyor portion:
i) is coupled to a line (7) to supply cells (C) for cellular therapy towards said conveyor portion (figures 1 to 4), or
ii) contains a charge comprising cells (C) for cellular treatment (figures 5 and 6)

Nevertheless, the invention also includes solutions in which such characteristics i) and ii) coexist, that is solutions in which the conveyor portion already contains a charge comprising cells for cellular therapy and at the same time is coupled to a line for supplying the cells for cellular therapy.

## Claims

1. Device for cellular therapy, said device being adopted of introduction through a catheter towards a site (V) subjected to treatment and including:
- an expandable portion (3; 10, 50) expandable between a contracted position and a deployed position at the treatment site (V), such as to apply angioplasty treatment to said site (V), and
- a conveyor portion (7, 8; 52) for receiving cells for cellular therapy (C) and of delivering said cells to said treatment site (V) while performing of said angioplasty operation, wherein said expandable portion comprises at least one balloon element (3) expandable between a contracted position and a deployed position at the treatment site and said conveyor portion comprises at least one opening (8) to diffuse cells (C) towards said treatment site (V), wherein said at least one balloon element (3) is provided on the external surface with grooves extending in a longitudinal direction of said balloon element (3), said at least one opening (8) to diffuse cells (C) towards said treatment site (V) is situated in an adjacent position to said at least one balloon element (3) **characterized in that** it includes an angioplasty stent (50) expandable between a contracted position and a deployed position at the treatment site, such as to perform an angioplasty treatment at said site, wherein said stent (50) possesses on its surface cavities (52) in the form of channels or grooves, and wherein said stent (50) includes, loaded on its external surface and in particular inside the cavities (52) provided on the external surface of, the stent, a charge comprising cells (C) for cellular therapy to deliver said cells to said treatment site.

2. Device according to claim 1, **characterised in that** said conveyor portion (7, 8; 52):
- is coupled to a line (7) for supplying cells (C) for cellular therapy towards said conveyor portion; and/or
- contains a charge comprising cells (C) for cellular therapy.

3. Device according to claim 1, **characterised in that** said expandable portion comprises a plurality of balloon elements (3) expandable between a contracted position and a deployed position at the treatment site.

4. Device according to claim 1 or claim 3, **characterised in that** said at least one balloon element (3) is situated at the distal extremity (2) of an introduction catheter.

5. Device according to any of the above claims, **characterised in that** said conveyor portion (8) is situated at the distal extremity (2) of an introduction catheter and comprises a tubular cavity (7) that extends along said catheter and constitutes a conduit for supplying cells (C) for cellular therapy towards said conveyor portion.

6. Device according to claim 1, **characterised in that**:
- said expandable portion comprises a plurality of balloon elements (3) expandable between a contracted position and a deployed position at the treatment site,
- said at least one opening (8) to diffuse cells (C) towards said treatment site (V) is situated in a zone between adjacent balloon elements (3) of said plurality of such elements.

7. Device according to claim 6, **characterised in that** openings (8) are provided to diffuse cells (C) towards said treatment site (V) only in at least one zone included between adjacent balloon elements (3) of said plurality of such elements.

8. Device according to claim 1, **characterised in that** said hollows (52) are provided on the radially external surface of said stent (50).

9. Device according to claim 1 or claim 8, **characterised in that** said cavities (52) are in the form of slots, notches, channels or grooves.

10. Device according to any of the claims from 1 to 9, **characterised in that** said stent (50) is mounted on the distal extremity of an introduction catheter (10).

11. Device according to claim 10, **characterised in that** said introduction catheter (10) is an expandable balloon catheter to produce expansion of the stent (50) from said contracted position to said deployed position.

12. Device according to claim 10, **characterised in that** said stent (50) is of the self-expanding type and said introduction catheter (10) presents a radial containment part to contain the stent that can be recalled to enable expansion of the stent (50) from said contracted position to said deployed position.

13. Device according to any of the claims from 1 to 12, **characterised in that** said stent (50) is provided with a coating of biocompatible carbonaceous material.

14. Device according to claim 13, **characterised in that** said coating of biocompatible carbonaceous material is present inside said cavities (52).

15. Device according to claim 13 or claim 14, **characterised in that** said coating of biocompatible carbonaceous material is present on the inner surface of said stent (50).

## Patentansprüche

1. Vorrichtung für die Zelltherapie, wobei die Vorrichtung für die Einführung durch einen Katheter zu einem Behandlungsort (V) ausgebildet ist und umfasst:
- einen aufweitbaren Bereich (3; 10; 50), der zwischen einer kontrahierten Position und einer entfalteten Position am Behandlungsort (V) aufweitbar ist, um eine Angioplastiebehandlung am Behandlungsort (V) durchzuführen, und
- einen Förderbereich (7, 8; 52), um Zellen für eine Zelltherapie (C) aufzunehmen und die Zellen dem Behandlungsort (V) während des Angioplastievorgangs zuzuführen, wobei der aufweitbare Bereich mindestens ein Ballonelement (3) umfasst, das zwischen einer kontrahierten Position und einer entfalteten Position am Behandlungsort aufweitbar ist, und der Förderbereich zumindest eine Öffnung (8) umfasst, um Zellen (C) in Richtung des Behandlungsorts (V) zu verteilen, wobei das mindestens eine Ballonelement (3) Rillen an der Außenoberfläche aufweist, die sich in der Längsrichtung des Ballonelements (3) erstrecken, und die mindestens eine Öffnung (8), um die Zellen (C) in Richtung des Behandlungsorts (V) zu verteilen, in benachbarter Position zu zumindest einem Ballonelement (3) angeordnet ist, **dadurch gekennzeichnet, dass** sie einen Stent (50) für Angioplastie umfasst, der zwischen einer kontrahierten Position und einer entfalteten Position am Behandlungsort aufweitbar ist, um eine Angioplastie am Behandlungsort durchzuführen, wobei der Stent (50) an seiner Oberfläche Ausnehmungen (52) in Form von Kanälen oder Rillen besitzt, und dass der Stent (50) an seiner Außenoberfläche und insbesondere in den Ausnehmungen (52), die an der Außenoberfläche des Stents vorgesehen sind, eine Charge mit Zellen (C) für die Zelltherapie umfasst, um die Zellen in dem Behandlungsort zuzuführen.

2. Vorrichtung nach Anspruch 1, **dadurch gekennzeichnet, dass** der Förderbereich (7, 8; 52):
- mit einer Leitung (7) zur Zuführung von Zellen (C) für die Zelltherapie in Richtung des Förderbereichs gekoppelt ist; und/oder
- eine Charge Zellen (C) für die Zelltherapie umfasst.

3. Vorrichtung nach Anspruch 1, **dadurch gekennzeichnet, dass** der aufweitbare Bereich eine Vielzahl von Ballonelementen (3) umfasst, die zwischen einer kontrahierten Position und einer entfalteten Position am Behandlungsort aufweitbar sind.

4. Vorrichtung nach Anspruch 1 oder 3, **dadurch gekennzeichnet, dass** zumindest ein Ballonelement (3) am distalen Ende (2) eines Einführungskatheters angeordnet ist.

5. Vorrichtung nach einem der vorherigen Ansprüche, **dadurch gekennzeichnet, dass** der Förderbereich (8) am distalen Ende (2) eines Einführungskatheters angeordnet ist und einen röhrenförmigen Hohlraum (7) umfasst, der sich entlang des Katheters erstreckt und einen Kanal zur Zuführung von Zellen (C) für die Zelltherapie in Richtung des Förderbereichs bildet.

6. Vorrichtung nach Anspruch 1, **dadurch gekennzeichnet, dass**:
- der aufweitbare Bereich eine Vielzahl von Ballonelementen (3) umfasst, die zwischen einer kontrahierten Position und einer entfalteten Position am Behandlungsort aufweitbar sind,
- zumindest eine Öffnung (8) zur Verteilung der Zellen (C) in Richtung des Behandlungsorts (V) in einer Zone zwischen benachbarten Ballonelementen (3) aus der Vielzahl dieser Elemente angeordnet ist.

7. Vorrichtung nach Anspruch 6, **dadurch gekennzeichnet, dass** Öffnungen (8) zum Verteilen der Zellen (C) in Richtung des Behandlungsorts (V) lediglich zumindest in einer Zone zwischen benachbarten Ballonelementen (3) aus der Vielzahl dieser Elemente vorhanden sind.

8. Vorrichtung nach Anspruch 1, **dadurch gekennzeichnet, dass** die Ausnehmungen (52) an der radial äußeren Fläche des Stents (50) angeordnet sind.

9. Vorrichtung nach Anspruch 1 oder 8, **dadurch gekennzeichnet, dass** die Ausnehmungen (52) eine Form von Schlitzen, Kerben, Kanälen oder Rillen aufweisen.

10. Vorrichtung nach einem der Ansprüche 1 bis 9, **dadurch gekennzeichnet, dass** der Stent (50) an dem distalen Ende eines Einführungskatheters (10) angebracht ist.

11. Vorrichtung nach Anspruch 10, **dadurch gekennzeichnet, dass** der Einführungskatheter (10) ein aufweitbarer Ballonkatheter ist, um die Aufweitung des Stents (50) von der kontrahierten Position zu der entfalteten Position zu erzeugen.

12. Vorrichtung nach Anspruch 10, **dadurch gekennzeichnet, dass** der Stent (50) von der selbst-expandierenden Art ist und dass der Einführungskatheter (10) einen radialen Aufnahmebereich zur Aufnahme des Stents aufweist, der abgerufen werden kann, um die Aufweitung des Stents (50) von der kontrahierten Position zu der entfalteten Position zu aktivieren.

13. Vorrichtung nach einem der Ansprüche 1 bis 12, **dadurch gekennzeichnet, dass** der Stent (50) eine Beschichtung aus einem bioverträglichen, kohlenstoffhaltigen Material aufweist.

14. Vorrichtung nach Anspruch 13, **dadurch gekennzeichnet, dass** die Beschichtung aus einem bioverträglichen, kohlenstoffhaltigen Material in den Ausnehmungen (52) ist.

15. Vorrichtung nach Anspruch 13 oder 14, **dadurch gekennzeichnet, dass** die Beschichtung aus einem bioverträglichen, kohlenstoffhaltigen Material auf der Innenfläche des Stents (50) ist.

## Revendications

1. Dispositif de thérapie cellulaire, ledit dispositif étant adapté pour l'introduction à travers un cathéter vers un site (V) soumis au traitement et comprenant :
une partie expansible (3 ; 10, 50) entre une position contractée et une position déployée au niveau du site de traitement (V), do manière à appliquer un traitement d'angioplastie audit site (V), et
une partie de transporteur (7, 8 ; 52) pour recevoir des cellules de thérapie cellulaire (C) et distribuer lesdites cellules audit site de traitement (V) tout en réalisant ladite opération d'angioplastie, où ladite partie extensible comprend au moins un élément de ballonnet (3) expansible entre une position contractée et une position déployée au niveau du site de traitement et ladite partie de transport comprend au moins une ouverture (8) pour faire diffuser des cellules (C) vers ledit site de traitement (V), où ledit au moins un élément de ballonnet (3) est muni sur selon la surface externe de rainures s'étendant dans une direction longitudinale dudit élément de ballonnet (3), ladite au moins une ouverture (8) pour faire diffuser des cellules (C) vers ledit site de traitement (V) est située dans une position adjacente audit au moins un élément de ballonnet (3) **caractérisé en ce qu'**il comprend une endoprothèse d'angioplastie (50) extensible entre une position contractée et une position déployée au niveau du site de traitement, de manière à réaliser un traitement d'angioplastie audit site, où ladite endoprothèse (50) possède sur sa surface des cavités (52) sous la forme de canaux ou rainures, et où ladite endoprothèse (50) comprend, chargée sur sa surface externe et en particulier à l'intérieur des cavités (52) prévues sur la surface externe de l'endoprothèse, une charge comprenant des cellules (C) de thérapie cellulaire pour remettre lesdites cellules audit site de traitement.

2. Dispositif selon la revendication 1, **caractérisé en ce que** ladite partie de transporteur (7, 8 ; 52) :
est couplée à une conduite (7) pour l'alimentation de cellules (C) de thérapie cellulaire vers ladite partie de transport ; et/ou
contient une charge comprenant des cellules (C) de thérapie cellulaire.

3. Dispositif selon la revendication 1, **caractérisé en ce que** ladite partie extensible comprend une pluralité d'éléments de ballonnet (3) expansible entre une position contractée et une position déployée au niveau du site de traitement.

4. Dispositif selon la revendication 1 ou la revendication 3, **caractérisé en ce que** ledit au moins un élément de ballonnet (3) est situé à l'extrémité distale (2) d'un cathéter d'introduction.

5. Dispositif selon l'une quelconque des revendications précédentes, **caractérisé en ce que** ladite partie de transport (8) est située à l'extrémité distale (2) d'un cathéter d'introduction et comprend une cavité tubulaire (7) qui s'étend le long dudit cathéter et constitue un conduit pour l'alimentation de cellules (C) de thérapie cellulaire vers ladite partie de transport.

6. Dispositif selon la revendication 1, **caractérisé en ce que** :
ladite partie extensible comprend une pluralité d'éléments de ballonnet (3) extensible entre une position contractée et une position déployée au niveau du site de traitement,
ladite au moins une ouverture (8) pour faire diffuser des cellules (C) vers ledit site de traitement (V) est située dans une zone entre des éléments de ballonnet adjacents (3) de ladite pluralité de ces éléments.

7. Dispositif selon la revendication 6, **caractérisé en ce que** des ouvertures (8) sont prévues pour faire diffuser des cellules (C) vers ledit site de traitement (V) uniquement dans au moins une zone incluse entre des éléments de ballonnet adjacents (3) de ladite pluralité de ces éléments.

8. Dispositif selon la revendication 1, **caractérisé en ce que** lesdites parties creuses (52) sont prévues sur la surface externe radiale de ladite endoprothèse (50).

9. Dispositif selon la revendication 1 ou la revendication 8, **caractérisé en ce que** lesdites cavités (52) sont sous la forme de fentes, d'encoches, de canaux ou de rainures.

10. Dispositif selon l'une quelconque des revendications 1 à 9, **caractérisé en ce que** ladite endoprothèse (50) est montée sur l'extrémité distale d'un cathéter d'introduction (10).

11. Dispositif selon la revendication 10, **caractérisé en ce que** ledit cathéter d'introduction (10) est un cathéter à ballonnet extensible pour produire l'expansion de l'endoprothèse (50) à partir de ladite position contractée vers ladite position déployée.

12. Dispositif selon la revendication 10, **caractérisé en ce que** ladite endoprothèse (50) est du type auto-extensible et ledit cathéter d'introduction (10) présente une partie de confinement radiale pour contenir l'endoprothèse, qui peut être rappelée pour permettre l'expansion de l'endoprothèse (50) à partir de ladite position contractée vers ladite position déployée.

13. Dispositif selon l'une quelconque des revendications 1 à 12, **caractérisé en ce que** ladite endoprothèse (50) est munie d'un revêtement de matériau carboné biocompatible.

14. Dispositif selon la revendication 13, **caractérisé en ce que** ledit revêtement de matériau carboné biocompatible est présent à l'intérieur desdites cavités (52).

15. Dispositif selon la revendication 13 ou la revendication 14, **caractérisé en ce que** ledit revêtement de matériau carboné biocompatible est présent sur la surface intérieure de ladite endoprothèse (50).
